# EUROPEAN PATENT APPLICATION

(11) **EP 4 015 035 A1**
(43) Date of publication of application: **22.06.2022**
(21) Application number: 21157912.3
(22) Date of filing: 18.02.2021
(51) Int. Cl.: A61N 1/372, A61B 6/12, A61B 6/00, A61B 90/00, A61B 5/11, A61B 5/00, G06T 7/00, A61N 1/05

(54) **STIMULATION LEAD ADHESION ESTIMATION**

(30) Priority: 15.12.2020 US 202063125905 P
(71) Applicant: Koninklijke Philips N.V., 5656 AG Eindhoven (NL)
(72) Inventor: PANSE, Ashish Sattyavrat, 5656 AE Eindhoven (NL); TOPOREK, Grzegorz Andrzej, 5656 AE Eindhoven (NL); FRANCIS, Nathan C, 5656 AE Eindhoven (NL); KRUECKER, Jochen, 5656 AE Eindhoven (NL); FLEXMAN, Molly Lara, 5656 AE Eindhoven (NL); SINHA, Ayushi, 5656 AE Eindhoven (NL); SHIMON, Jeff, 5656 AE Eindhoven (NL); SALEHI, Leili, 5656 AE Eindhoven (NL); ERKAMP, Ramon Quido, 5656 AE Eindhoven (NL)
(74) Representative: Philips Intellectual Property & Standards

(57) **Abstract**

A method is provided for determining whether a section of a lead has adhered to a blood vessel. The method comprises obtaining data corresponding to the blood vessel with a lead inside and determining motion vectors corresponding to the lead from the data corresponding to the blood vessel. The motion vectors are input into a machine learning algorithm trained to learn the correlation between the motion vectors and whether a section of a lead has adhered to a blood vessel and output an adherence level for segments of the lead.

## Description

### TECHNICAL FIELD

The present disclosure relates to the field of removing implanted cardiac devices. In particular, the present disclosure relates to estimating whether the leads of the cardiac device have adhered to the walls of a blood vessel.

### BACKGROUND

The extraction of a surgically implanted cardiac device, such as a pacemaker or a defibrillator, may be required under certain conditions, such as in cases of malfunction or infection. The removal of such devices may potentially imply a high-risk procedure.

Particularly, a wire or wires, commonly referred to as leads, typically run from a control unit, e.g. a pulse signal generator, to the heart. In a pacemaker, these leads enable the device to influence the heart rate by delivering a train of electric pulses, while in a defibrillator, the lead may be adapted for delivering a high-energy pulse to the heart to disrupt a dangerous fibrillation condition.

The lead typically runs through a vein to the heart. The tip of the lead may typically be anchored to the heart muscle, e.g. by tines, a screw or hook and, after a few months after implantation, scar tissue.

The extraction of pacemaker or defibrillator leads can be performed by various tools including simple sheaths, a laser-based extraction device or a rotating mechanical cutting device.

More generally, most cardiovascular implantable electronic devices (CIEDs) currently use leads that connect the generator to cardiac tissue. Lead management is an important issue, given the lead failures, generator changes, and clinical conditions that can directly affect CIEDs, such as infection.

Approximately 1.5 million implanted cardiac devices are placed annually. Although recent technological advances have eliminated the need for transvenous or epicardial leads for CIEDs used in select groups, lead management remains critical for a variety of reasons including malfunction or recall of a pacemaker or ICD lead, device upgrade or revision, venous occlusion, implant site (pocket) infection, blood stream infection, MRI contraindication due to the presence of an abandoned lead or patient desire to have a system removed because of personal preference or no further indication.

Cardiac CT imaging is typically performed to assess vascular or extracardiac lead positioning and potentially identify sites of venous adhesions. Fluoroscopy is typically used to identify sites of venous occlusion or stenosis and assess regions of lead mobility and adherence.

Lead extraction is carried out with tools like Spectranetics' GuideLight and TightRail which go over the leads, and separate them from the vessels and scar tissue using a mechanical cutting tip or a laser cutting tip. During the procedure, the physicians rely on tactile feedback from the device to determine lead adherence.

The preoperative CT used to determine lead adhesion is affected by metal artifacts, which reduce the diagnostic accuracy of determining intravascular lead positioning. During lead extraction procedures, there is a risk of vessel tear especially in the vicinity of the superior vena cava (SVC). This could be avoided if the physician can get more information about the adhesion during the extraction procedure.

Thus, there is a need for an improved method of determining the adhesion of a lead inside a blood vessel. Additionally, there is a need for a method of determining the adhesion level of a lead inside a blood vessel during an extraction operation.

### SUMMARY

Aspects of the present disclosure provide devices, system, methods and computer programs for determining whether a section of a lead has adhered to a blood vessel and are defined invention by the claims.

According to examples in accordance with an aspect of the present disclosure, there is provided a method for determining whether a section of a lead has adhered to a blood vessel, the method comprising:
receiving or obtaining data corresponding to the blood vessel, wherein the blood vessel has a lead inside;
determining motion vectors corresponding to the lead from the data corresponding to the blood vessel;
inputting the motion vectors into a machine learning algorithm, wherein:
   the machine learning algorithm is trained to learn the correlation between the motion vectors and whether a section of a lead has adhered to a blood vessel, and
   the machine learning algorithm is configured to output an adherence level for segments of the lead based on the input motion vectors.

The receiving or obtaining of data may be done using the input interface while outputting of result data may be done using the output interface. The determination and processing steps may be done using a processor as described herein. The method may be a computer implemented method.

It is known that by observing the lead moving inside a blood vessel, a user can estimate where the lead has adhered to the blood vessel or not. However, this requires experience and knowledge of the different viewing angles used to view the moving lead. In some instances, the lead may be moving transversely to the viewing plane and thus the movement cannot be seen.

The data corresponding to the blood vessel preferably contains images of the blood vessel. Alternatively (or additionally), the data may contain information of the blood vessel and the lead inside obtained via Fiber Optic RealShape (FORS), electromagnetic tracking or similar methods.

Images of blood vessels with a lead inside contain a lot of information relating to the blood vessel, the tissue near the blood vessel and the lead. If two images are obtained at different times, the two images will be different due to the movements of blood vessel, the tissue and the lead with time.

The changes between the images can be quantified by motion vectors. In fact, the motion vectors also relate to the movements made by the objects in the image (blood vessel, tissue and lead).

The inventors have realized that the motion vectors contain all the information required regarding the movement of the lead, whilst removing unnecessary information in the images (e.g. colors, shapes etc.). Thus, a machine learning algorithm can be trained effectively to learn the patterns between the motion vectors and whether the lead has adhered to the wall of a blood vessel.

The machine learning algorithm outputs an adherence level (or level of adherence) for segments of the lead. In other words, the adherence level corresponds to the likelihood of a segment of the lead to be adhered to the blood vessel. In some instances, the adherence level may be a binary classification (i.e. yes/no). However, in other instances, the adherence level may indicate a probability of adherence, or in another example, the adherence level may indicate the severity of the adherence, or level-of calcification of the adherence.

Adherence and adhesion are used interchangeably in this context, referring to the undesirable physical connection between the lead and the blood vessel.

The segments of the lead refer to an area (or volume) of a pre-defined size which fully contain the area (or volume) of the lead visible in images. For example, the segments may be a single pixel or a group of 10x10 pixels. Alternatively, the segments may contain a single motion vector each (or more than one). The size of the segments is arbitrary and may depend on the processing resources available and/or the particular training of the machine learning algorithm.

The motion vectors may be 2D motion vectors. 2D motion vector can have a vectorial and/or non-vectorial parametrization form. The parametrizations can be in form of x-/y-translation, direction and a magnitude for the translation, quaternions, matrix, exponential map, vector fields.

The inventors have realized that the motion in 2D of the lead correlates to the adhesion of a lead to the blood vessel. Thus, the inventors trained a machine learning algorithm to learn the correlation between the motion in 2D and the level of adhesion between the lead and the blood vessel. The motion in 2D is given by 2D motion vectors which are determined from images of the blood vessel.

Using a machine learning algorithm with 2D motion vectors as inputs allows a user to determine whether a lead has adhered to a blood vessel without the strict need of 3D imaging modalities. The 2D motion vectors can be determined from 2D, 3D or 4D (i.e. time dependent) imaging.

Additionally, by inputting the 2D motion vectors into the machine learning algorithm, there may be no need to input the full images. This reduces the overall data which is input into the machine learning algorithm, thus improving the speed at which the adherence level is determined. This could, for example, allow the determination of adherence level to be done in real time and/or allow an increase in complexity of the machine learning algorithm (which could improve accuracy).

The images of the blood vessel may be 2D images.

Determining whether a lead has adhered to a blood vessel is typically done with 3D imaging modalities, such as CT scanning. Thus, the movement of the lead and the blood vessel can be visualized in 3D space and the user can determine whether there is adherence of the lead to the blood vessel. However, this requires 3D imaging and analysis in 3D space which is difficult to visualize and is time consuming to analyze.

The inventors have realized that motion vectors obtained from 2D images also correlate to the adherence level of the wall of a blood vessel. Thus, 3D imaging modalities are not required and the workflow of obtaining the images can be reduced.

Obtaining images of the blood vessel may be based on 2D fluoroscopy and/or 2D venography.

Fluoroscopy and/or venography are typically used during an operation for removing leads from a subject (i.e. intra-op). Thus, the images obtained from these imaging modalities can be used to determine the level of adhesion without the need of imaging before the operation during an operation.

Further inputs to the machine learning algorithm may be the images of the blood vessel. The machine learning algorithm can also incorporate x-ray images as inputs. X-ray images (or roadmap images such as venograms) contain additional information about the blood vessel and/or any anatomical motion, which could be used by the machine learning algorithm to find useful correlations between the lead and vessel motion.

For example, there are multiple sets of inputs possible: motion vectors only (preferred) or motion vectors and the current or sequence of images (e.g. combination of fluoroscopy and venogram images).

Alternatively, the machine learning algorithm may comprise a motion vector layer for determining the motion vectors from, for example, venogram images only. The machine learning algorithm could then determine the motion vectors and determine the adhesion level from said motion vectors.

The machine learning algorithm may be further configured to output a position of adhesion based on the segments and their respective adherence level. Localization of the adhesion is an important aspect of determining the adhesion level. As the segments implicitly have relative locations to each other, the locations of the adhesion (e.g. over 3 segments) may could also be output by the machine learning algorithm as a position of adhesion. Alternatively, the machine learning algorithm may output the segments with an adhesion level indicating adhesion on an image of the blood vessel, thus indicating the position of the adhesion on the blood vessel.

The machine learning algorithm may be further configured to output a suggested viewing angle, wherein an image of the blood vessel taken at the suggested viewing angle is likely to provide accurate motion vectors.

The machine learning algorithm may be based on:
a convolutional neural network;
a multi-layer perceptron;
a support vector machine;
a decision tree model;
multivariate regression model; or
a combination thereof.

The method may further comprise displaying the adherence level with the corresponding motion vectors on the images of the blood vessel.

The adherence level may be based on a binary classification or an adherence range.

The present disclosure also provides a computer program product comprising computer program code means which, when executed on a computing device having a processing system, cause the processing system to perform all of the steps of the method for determining whether a section of a lead has adhered to a blood vessel

The present disclosure also provides a system for determining whether a section of a lead has adhered to a blood vessel, the system comprising:
an input interface configured to receive or obtain data corresponding to the blood vessel, wherein the blood vessel has a lead inside; and
a processor configured to:
   determine motion vectors corresponding to the lead for the data corresponding to the blood vessel;
   input the motion vectors into a machine learning algorithm, wherein:
      the machine learning algorithm is trained to learn the correlation between the motion vectors and whether a section of a lead has adhered to a blood vessel, and
      the machine learning algorithm is configured to output an adherence level for segments of the lead.

The input interface is communicatively coupled to the processor which in turn is communicatively coupled to the output interface.

The system may further comprise an imaging device configured to obtain images of the blood vessel and send the images to the interface.

The imaging device may be a 2D imaging device configured to obtain 2D images.

The imaging device may be configured to obtain 2D fluoroscopic images and/or 2D venography images.

The system may further comprise a user interface for providing the adherence level to a user in visible and/or audible format. A preferred user interface comprises a display configured to display at least the adherence level and preferably together with images of the blood vessel, the motion vectors and/or the corresponding adherence level.

The present disclosure also provides a method for training a machine learning algorithm to learn the correlation between motion vectors corresponding to a lead inside a blood vessel and whether a section of the lead has adhered to the blood vessel, the method comprising:
obtaining a first set of data corresponding to a blood vessel with a lead inside;
determining a ground truth for the first set of data corresponding to the blood vessel, wherein the ground truth defines whether the lead has adhered to the blood vessel and the location of the adhesion;
obtaining a second set of data corresponding to the blood vessel with a lead inside, wherein the second set of data is obtained at the same anatomical locations as the first set of data within the blood vessel;
determining motion vectors corresponding to the lead from the second set of data corresponding to the blood vessel;
inputting the motion vectors into the machine learning algorithm, wherein the machine learning algorithm is configured to output an adherence level for segments of the lead;
comparing the adherence levels output by the machine learning algorithm to the corresponding ground truth and determining an error based on the comparison between the adherence levels and the ground truth; and
adapting parameters of the machine learning algorithm based on the error.

The first set of data may be CT images as it is relatively simple (compared to other data modalities) to obtain an accurate ground truth. The second set of data may be 2D X-ray images or venograms. Alternatively, both the first set and second set may be the same set of 2D X-ray images (or the same set of a different data type). The second set of data will be the data type which the machine learning algorithm will learn from. The first set of data is only for determining the ground truth.

The first set of data may be obtained from lead extraction procedures. The ground truth may be obtained by annotating data from lead extraction procedures (i.e. lead adhesion encountered (yes/no) for different segments along the lead).

The first set of data may be obtained from spatial sensing technology, such as electromagnetic tracking or FORS, from previous lead extraction procedures.

The first set of data may be obtained from visual inspection of previous lead extraction procedures.

Comparing the adherence levels to the ground truth may be based on a cross-entropy loss function (typically used for binary classification tasks) or by performing a stochastic gradient decent.

The parameters of the machine learning algorithm may be a set of weights which map the inputs of the machine learning algorithm to the outputs of the machine learning algorithm.

These and other aspects of the present disclosure will be apparent from and elucidated with reference to the embodiment(s) described hereinafter.

### BRIEF DESCRIPTION OF THE DRAWINGS

Illustrative embodiments and examples of the present disclosure will be described with reference to the accompanying schematic Figures, , of which:
Figure 1 shows an image of a blood vessel with a previously implanted cardiac lead inside the blood vessel;
Figure 2 shows an image of the blood vessel with a lead inside and the motion vectors corresponding to the lead;
Figure 3 shows the inputs and outputs of a machine learning algorithm for determining an adherence level of the lead;
Figure 4 shows an image of the blood vessel with the segments output by the machine learning algorithm;
Figure 5 shows an example architecture of the machine learning algorithm;
Figure 6 shows an example of the training data used on the machine learning algorithm;
Figure 7 shows CT images of a blood vessel with two leads used for training; and
Figure 8 shows an example training method for the machine learning algorithm.
Figure 9 shows a diagram of a processing system as defined according to embodiment of the present disclosure.
Figure 10 shows an imaging system comprising the processing system as described herein such as for example with respect to Figure 9.

### DETAILED DESCRIPTION OF THE EMBODIMENTS

For a better understanding of the principles of the present disclosure, and to show more clearly how it may be carried into effect, reference will now be made, by way of example only, to the embodiments described with reference to the accompanying drawings.

The detailed description and specific examples, while indicating exemplary embodiments of the apparatus, systems and methods, are intended for purposes of illustration only and are not intended to limit the scope of the present disclosure. These and other features, aspects, and advantages of the apparatus, systems and methods of the present disclosure will become better understood from the following description, appended claims, and accompanying drawings. The Figures are merely schematic and are not drawn to scale. The same reference numerals are used throughout the Figures to indicate the same or similar parts.

The present disclosure provides a method for determining whether a section of a lead has adhered to a blood vessel, e.g. a section of the inner wall of the blood vessel. The method comprises obtaining data corresponding to the blood vessel with a lead inside (preferably images of the blood vessel) and determining motion vectors corresponding to the lead from the data corresponding to the blood vessel. The motion vectors are input into a machine learning algorithm trained to learn the correlation between the motion vectors and whether a section of a lead has adhered to a blood vessel and output an adherence level for segments of the lead.

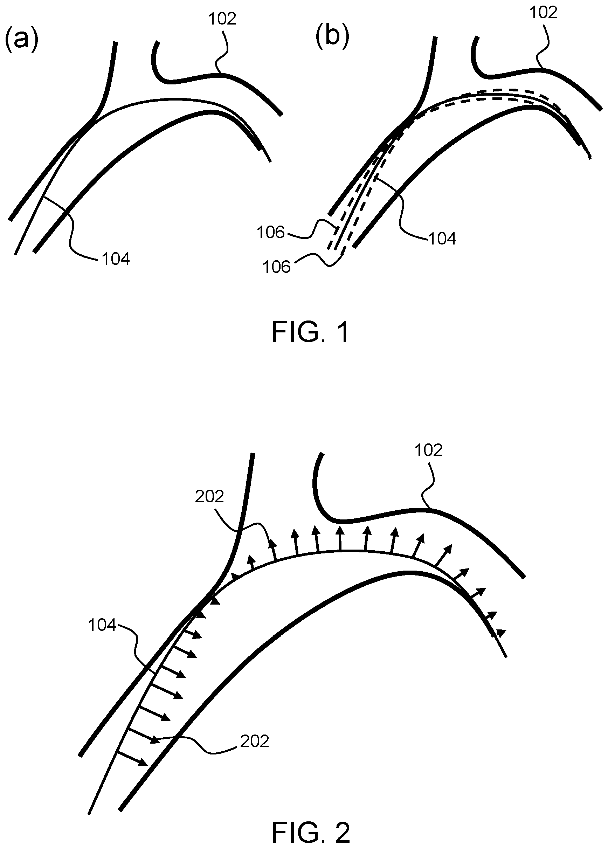

Figure 1 shows an image of a blood vessel 102 with a previously implanted cardiac lead 104 inside the blood vessel 102. Figure 1 (a) shows an image of the blood vessel 102 with the lead 104 and Figure 1 (b) shows an image of the blood vessel 102 with the possible movements of the lead 104 within the blood vessel 102.

The dashed lines 106 in Figure 1 (b) show the extremes of where the lead 104 can move within the blood vessel 102. This can be observed, for example, in x-ray imaging or CT scans of the blood vessel 102. The movement of the lead 104 may be due to, for example, the heart beat of the subject, the respiration of the subject or the blood flow in the blood vessel. The motion may also be induced by peri-operative interactions with the lead 104 such as for example those caused by manipulation of parts during a procedure performed by a physician.

The blood vessel 102 also has a motion range (not shown). In the case where the lead 104 is not adhered to the walls of the blood vessel 102, the motion of the lead 104 has very little correlation with the motion of the blood vessel 102. Figure 1 shows two regions where the lead has adhered to the wall of the blood vessel 102. In this case, the lead 104 moves as the blood vessel 102 moves. In the other segments where the lead 104 is not attached to the walls of the blood vessel 102, the motion of the lead 104 is a more complex motion. This range of motion is defined by the dashed lines 106 around the lead 104 in Figure 1 (b).

If the lead 104 has adhered to the walls of the blood vessel 102, it is unlikely that any movement relative to the blood vessel 102 will be observed at the points where the lead 104 has adhered to the blood vessel 102. The movement of the lead 104 within the blood vessel 102 can be quantified by using motion vectors.

Figure 2 shows an image of the blood vessel 102 with a lead 104 inside and the motion vectors 202 corresponding to the lead 104. The motion vectors 202 are vectors which depict the direction and magnitude of overall motion of the lead 104 within the blood vessel 102. In Figure 2 the blood vessel 102 is depicted as motionless, however, in some cases, it may be necessary to determine the motion vectors 202 relative to the blood vessel by, for example, also determining the motion vectors of the blood vessel 102.

The lead 104 may be identified in the images using, for example, thresholding, multi-scale ridge detection methods (or their variations such as hessian-based vesselness filters), active contours methods or deep learning based methods such as segmentation (e.g. U-net architecture). Other methods for identifying the lead 104 will be known to a person skilled in the art.

In an example, the implanted leads 104 are visible in fluoroscopic images and in some cases, thicker vessel walls are also visible. Motion vectors 202 in relation to the blood vessel 102 could be derived from fluoroscopic images with contrast in the blood vessels 102. The motion vectors 202 can be derived from fluoroscopic image sequences using pixel based (i.e. direct) or feature based (i.e. indirect) motion vector estimation methods. Deep learning based methods can also be used to compute motion vectors. Methods for determining motion vectors 202 will be known to a person skilled in the art.

Examples are for example discussed in the articles "Feature Based Methods for Structure Motion Estimation" of P.H.S. Torr and A. Zisserman and "All About Direct Methods" of M. Irani and P. Anandan (both available at www.robots.ox.ac.uk).

The lead images could alternatively be derived from a sensing technology such as electromagnetic sensing, Fiber Optic RealShape (FORS), or similar techniques.

Alternatively, the motion vectors 202 for the lead 104 can be derived in 3D by using 2D fluoroscopic images acquired from multiple angles. In a typical lead extraction case, 2D fluoroscopic images are acquired from multiple angles to visualize the curvature of the leads 104. The 2D fluoroscopic images can be used to reconstruct the lead 104 in 3D by back-projecting the segmented leads 104 from the 2D fluoroscopic images. Using 3D motion vectors provides more information to a machine learning algorithm to classify the level of adhesion. 3D motion vectors could also be obtained from CT scans (i.e. 4D CT - sequence of 3D images in time).

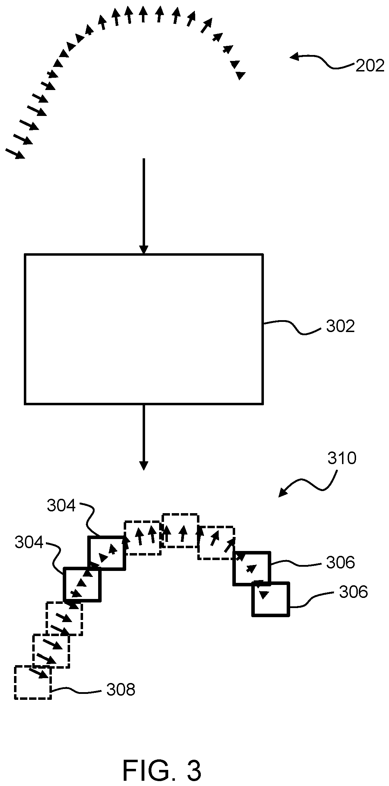

Figure 3 shows the inputs and outputs of a machine learning algorithm 302 for determining an adherence level 310 of the lead 104. The motion vectors 202 of the lead 104 are the inputs into the machine learning algorithm 302. The machine learning algorithm 302 has been trained to learn the patterns/correlation between the motion vectors 202 of a lead 104 and whether the lead 104 has adhered to the wall of a blood vessel 102. The machine learning algorithm 302 then outputs an adherence level 310 for the lead 104.

Motion vectors for the blood vessel 102 may also be input to the machine learning algorithm 302. Alternatively (or additionally), images of the blood vessel or venograms of the blood vessel may be input. These would provide the machine learning algorithm with knowledge of the movement of the blood vessel 102.

In Figure 3 the adherence level 310 is shown as a binary choice for different segments 304, 306, 308 of the lead 104. The segments 304, 306, 308 could be, for example, based on a number of motion vectors 202 within the segment or based on a pre-defined size. The machine learning algorithm 302 outputs segments 304 and 306 as being "adhered" and the rest of the segments 308 (in dashed lines) as "not adhered".

Alternatively, the machine learning algorithm 302 could output a range of probabilities for each segment 304, 306, 308. Additionally, the machine learning algorithm 302 could output the same motion vectors 202 as were input, but with an additional adherence level variable for each motion vector 202. For example, the machine learning algorithm 302 could output the motion vectors 202 with different colors (e.g. green defining "not adhered" and orange defining "adhered").

Alternatively, the visualization of the adherence level 310 can be a color gradient along the lead 104 showing the probability of all segments 304, 306, 308 of the lead 104 being adhered to the wall of a blood vessel 102. The visualization could also be based on the location of the tip of the therapy device over the lead 104 and which zone it is within (i.e. only visualizing certain parts of the lead 104 to avoid unnecessary clutter). It may be advantageous to only visualize the adherence levels (color-codding) when the therapeutic device, such as mechanical atherectomy device, is in the vicinity of the adhesion as this may reduce the information clutter.

The size of the segments 304, 306, 308 could be the size of a pixel (dependent on resolution) or a pre-defined number of pixels wide/tall.

In short, the machine learning algorithm 302 is configured to identify segments 304, 306, 308 of a lead 104 which have adhered to the wall of a blood vessel 102. This can be used to avoid vessel rupture during lead extraction, select between laser or mechanical lead extraction device and/or speed up the lead extraction procedure.

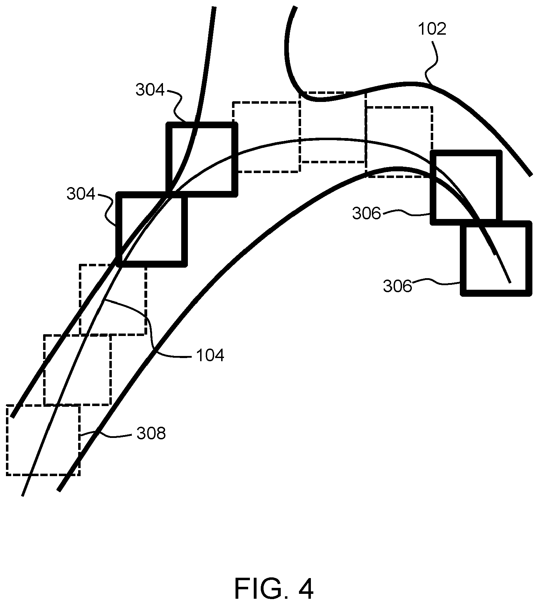

Figure 4 shows an image of the blood vessel 102 with the segments 304, 306, 308 output by the machine learning algorithm 302. The segments 304 and 306 with the "adhered" adherence level are shown on/near points of the lead 104 which are in touch with the wall of the blood vessel 102, thus identifying them as being adhered.

The output of the machine learning algorithm 302 can be overlaid on the images of the blood vessel 102 (e.g. the original images used to determine the motion vectors 202) in order to give the user an idea of where the lead 104 may be attached to the wall of the blood vessel 102. The motion vectors 202 could also be displayed.

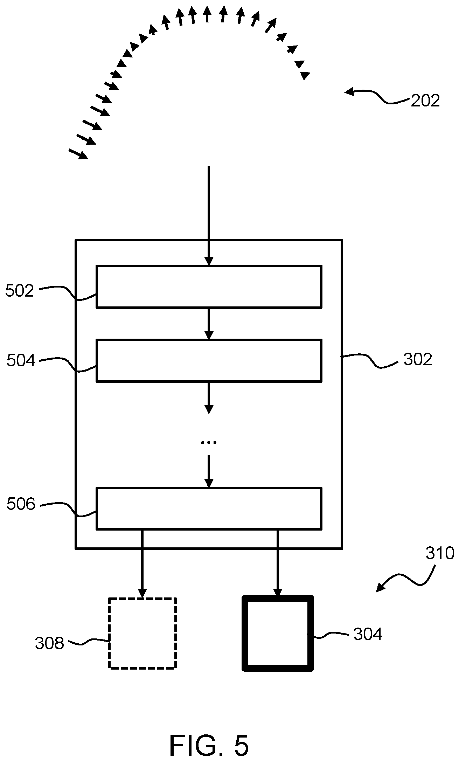

Figure 5 shows an example architecture of the machine learning algorithm 302. The inputs 502 to the machine learning algorithm 302 are the motion vectors 202 of the lead 104 embedded in the blood vessel 102 and the outputs are a binary indication of the adherence level 310 for segments 304, 306, 308 along the lead 104. The machine learning algorithm 302 is a convolutional neural network (CNN) with Softmax activation function as the last layer 506. The loss function for the Softmax used is the log loss function. In other examples, activation function can be a sigmoid or other non-linear activation function, especially when network performs a regression task, for instance, predicts the severity or level of calcification of the adherence. A rectified linear unit (ReLU) function may e.g. be used.

The number of interim layers 504, 506 will depend on the particular implementation used. For example, it may depend on the functions used for each layer, the total time from input to output (the CNN may be used in real time) or particular design choices of the designer of the CNN. The CNN will typically consist of three or more interim layers 504, 506, each layer consisting of convolution operation, batch normalization, dropout, pooling operation, and/or non-linear activation.

The motion vectors 202 from, for example, a venogram or fluoroscopic images are calculated and input to the CNN. The CNN outputs a binary classification the segments 304, 306, 308 along the lead 104 as adhered or non-adhered. Classes are determined from logits generated by the CNN that are passed to an activation function (e.g. sigmoid) that maps them to values between 0 and 1. A threshold (e.g. 0.5) can then be used to determine the class.

Alternatively, the adherence level 310 output by the CNN could be a color gradient along the lead 104 showing values generated by the CNN in the range [0,1] (e.g. smooth color-map). These values may be interpreted roughly as the probability of each point on the lead being adhered to the wall of a blood vessel 102.

A CNN that uses only venograms could also be used. Here, the venogram frames as well as the motion vectors 202 would be input into the CNN. As the blood vessels 102 are visible in the venograms, not only can motion vectors 202 be extracted from the blood vessels 102, but the CNN can also learn from observing whether the leads 104 are surrounded by tissue to aid its predictions. Therefore, the CNN learns clues from both the vessel/lead motions and anatomical features to predict adhesion of the lead 104. The anatomical context is particularly useful as, for instance, it is well known that, in most subjects, the lead 104 is adhered to the wall of a blood vessel 102 near the subclavian vein.

Alternatively, a CNN that uses only unidirectional non-contrast enhanced fluoroscopic images could be used. Image sequences could be taken once at a lower heart rate and after at an increased heart rate induced by exercise, medication, or a pacing device rate modification. The motion vectors 202 corresponding to non-adhered areas would show more pattern change than in the adhered regions.

The machine learning algorithm 302 could, alternatively, be a multi-layer perceptron (MLP), a support vector machine (SVM), a decision tree (random forest) model, a multivariate regression (linear or logistic regression), or a combination thereof.

Additionally, the machine learning algorithm 302 could also output optimum viewing angles in order to maximize the apparent motion of the lead 104. This can be achieved by a machine learning algorithm 302 which has been trained by observing multiple motion vectors 202 of the same lead 104 at different viewing angles. The machine learning algorithm 302 could then output a viewing angle suggestion from which the user could obtain further motion vectors 202.

An example system which uses said machine learning algorithm 302 could consist of an interventional x-ray system with an x-ray tube adapted to generate x-rays and an x-ray detector configured to acquire x-ray images. Examples of such x-ray systems are fixed c-arm x-ray systems and mobile x-ray systems.

A processor is then used to calculate the motion vectors 202 along the length of the lead 104 to be extracted from thee x-ray images. The motion vectors 202 can be determined at different times (e.g. for each new frame/set of frames) and along the length of the lead 104.

The processor then inputs the motion vectors 202 to a machine learning algorithm 302 that is configured classify segments 304, 306, 308 along the lead as, for example, adhesion pockets 304, 306 and un-adhered pockets 208.

### TRAINING THE MACHINE LEARNING ALGORITHM

The training data used to train the machine learning algorithm 302 consists of motion vectors 202 of the implanted lead 104, motion vectors of blood vessels 102 in which the leads 104 are commonly implanted and the ground truth (i.e. is the lead 104 adhered to the blood vessel 102 for each segment 304, 306, 208). The motion vectors 202 can be derived from time series x-ray images. The ground truth can be obtained from pre-operational CT images.

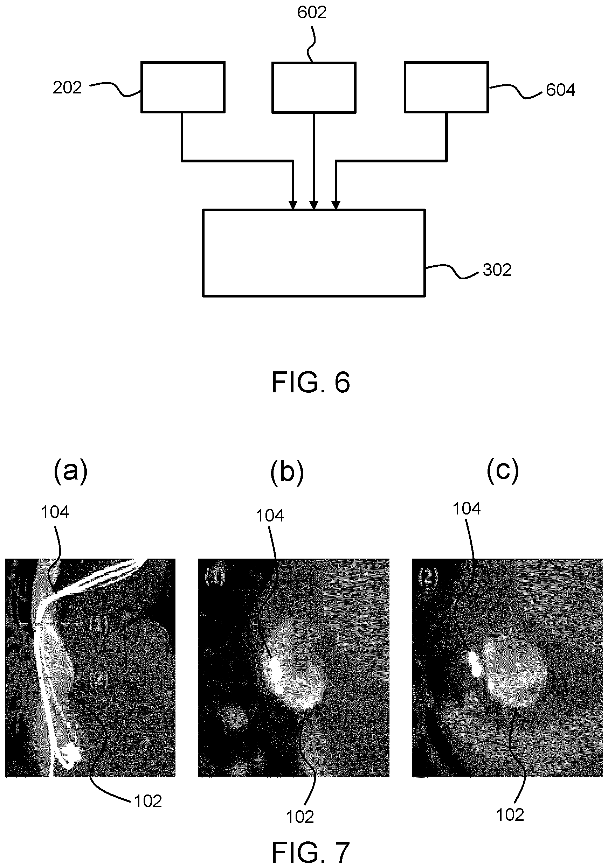

Figure 6 shows an example of the training data 202, 602, 604 used on the machine learning algorithm 302. The training is carried out on dataset tuples described by di = (m, mv, G) where:
m: motion vectors 202 of the lead 104;
mv: motion vectors of the vessel 102; and
G: ground truth 604 of adhesion (or non-adhesion).

Figure 7 shows CT images of a blood vessel 102 with two leads 104. The images can be used to identify the ground truth 604 from the CT images. Figure 7 (a) shows a CT image of the blood vessel 102 and Figures 7 (b) and 7 (c) show cross sections (1) and (2) of Figure 7 (a).

The cross section at the region (1) is shown in Figure 7 (b) where the leads 104 are free from the wall of the blood vessel 102 (i.e. not adhered). In region (2), Figure 7 (c), the leads 104 appear outside the contrast filled blood vessel 102, thus showing that adhesion has occurred. Thus, the ground truth can be obtained from the CT images.

Alternatively, the ground truth 604 on adhesion of the lead 104 can be obtained by annotating data from lead extraction procedures (lead adhesion encountered (yes/no) for different segments along the lead 104). The location of adherence can be logged automatically if the lead extraction device is equipped with a spatial sensing technology such as electromagnetic, FORS etc. In addition, it can be logged whether a given lead extraction device was successful in overcoming adhesion of the lead 104 to the blood vessel 102. This information can be used to train a separate model to predict whether a given device with leads 104 will be successful in other procedures.

Another method for obtaining ground truth 604 on lead adhesion is visual inspection of the lead 104 after removal. One can measure the distances from the tip to where tissue has adhered to the lead 104. A CT scan can provide the 3D shape, and its registration to, for example, the fluoroscopic images allows localization of the adhesion.

Alternatively, the machine learning algorithm 302 can be trained in an end-to-end fashion. For instance, the inputs to the machine learning algorithm 302 could be a sequence of sections (ROI) along the lead 104. Each section would cover both the blood vessel 102 and the lead 104, thus implicitly containing the relative motion between lead 104 and wall of the blood vessel 102. Given this input, a sequence neural network could be trained to predict the presence or absence of adhesion of the lead 104 to the blood vessel 102.

The machine learning algorithm 302 may comprise a motion vector layer trained to determine motion vectors 202 from images of the blood vessel 102 and determine the adhesion level based on the motion vectors.

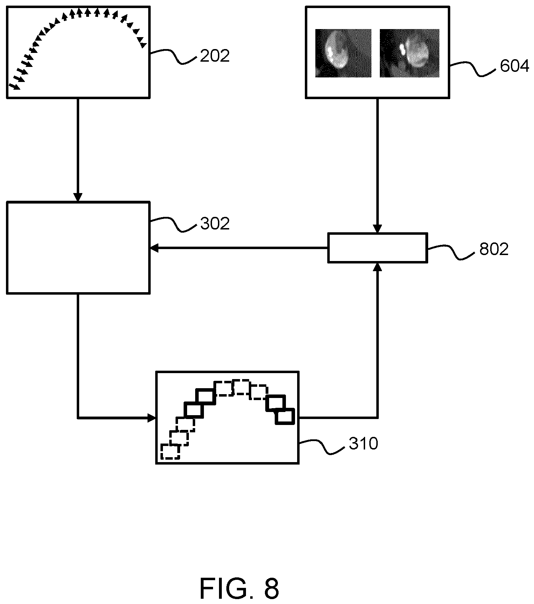

Figure 8 shows an example training method for the machine learning algorithm 302. A selected lead 104 from an image (e.g. fluoroscopic, cine, or venogram) is divided into smaller segments 304, 306, 308 and the motion vectors 202 are calculated for each segments along the lead 103. The segments can range from a pixel to few pixels. Motion vectors 202 are calculated for each of the segments. These are used to train a machine learning algorithm 302 which compares 802 its output adherence level 310 to the ground truth 604 obtained from the corresponding pre-operational CT images. The ground truth 604 locations of adhesions are determined from the CT images and the corresponding segments are assigned a label as adhered or not adhered.

The parameters of the machine learning algorithm 302 are iteratively optimized during the training process, by comparing 802 output adherence levels 310 with the ground truth 604 using, preferably, a cross-entropy loss function (typically used for binary classification tasks) and performing stochastic gradient decent. Other loss functions could also be used.

A machine-learning algorithm 302 is any self-training algorithm that processes input data in order to produce or predict output data. Here, the input data comprises motion vectors and the output data comprises an adherence level.

Suitable machine-learning algorithms for being employed in the present disclosure will be apparent to the skilled person. Examples of suitable machine-learning algorithms include decision tree algorithms and artificial neural networks. Other machine-learning algorithms such as logistic regression, support vector machines or Naive Bayesian models are suitable alternatives.

The structure of an artificial neural network (or, simply, neural network) is inspired by the human brain. Neural networks are comprised of layers, each layer comprising a plurality of neurons. Each neuron comprises a mathematical operation. In particular, each neuron may comprise a different weighted combination of a single type of transformation (e.g. the same type of transformation, sigmoid etc. but with different weightings). In the process of processing input data, the mathematical operation of each neuron is performed on the input data to produce a numerical output, and the outputs of each layer in the neural network are fed into the next layer sequentially. The final layer provides the output.

Methods of training a machine-learning algorithm are well known. Typically, such methods comprise obtaining a training dataset, comprising training input data entries and corresponding training output data entries. An initialized machine-learning algorithm is applied to each input data entry to generate predicted output data entries. An error between the predicted output data entries and corresponding training output data entries is used to modify the machine-learning algorithm. This process can be repeated until the error converges, and the predicted output data entries are sufficiently similar (e.g. ±1%) to the training output data entries. This is commonly known as a supervised learning technique.

For example, where the machine-learning algorithm is formed from a neural network, (weightings of) the mathematical operation of each neuron may be modified until the error converges. Known methods of modifying a neural network include gradient descent, backpropagation algorithms and so on.

A single processor or other unit may fulfill the functions of several items recited in the claims.

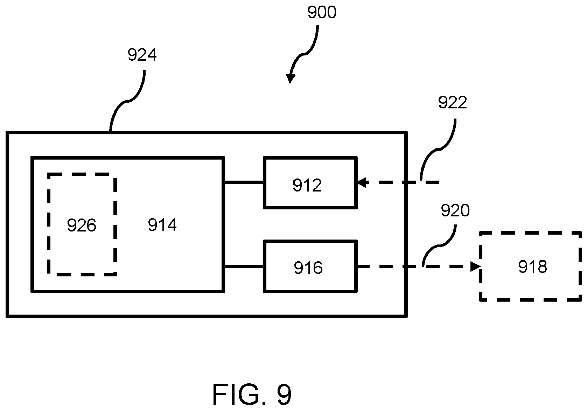

Figure. 9 is a schematic diagram of a system 900 for determining whether a section of a lead (104) has adhered to a blood vessel (102). The system may be processing system or unit. The system may take the form of a computer, controller or workstation in stand-alone or integrated fashion and having appropriate hardware and software for performing the purpose indicated. In integrated form, the system may be part of or integrated in an imaging system such as for example an X-ray or CT imaging system. The system includes a data processor and appropriate data inputs and outputs according to embodiments described herein to carry out one or more steps of methods described herein. The data processor may be a single processor, but alternatively a distributed processor may be used in which two or more processing units are distributed over multiple devices or locations and configured to be able to communicate with each other. Such communication may for example occur through wireless or wired communication connections and devices with or without making use of communications networks.

The system 900 comprises an input interface 912, a data processor 914 and an output interface 916 and may comprise a memory 926. The input interface 912 is configured to receive or obtain data corresponding to the blood vessel (102), wherein the blood vessel (102) has a lead (104) inside. For example, the input interface may comprise an image data input interface configured to receive or obtain such data comprising image data (as described herein above) of such vessel having the lead inside. One example of data are 2D or 3D X-ray image data. The input of data is indicated with a hashed arrow 922 and the input may be connected to an output of image data of an imaging device.

The data processor 914 is communicatively coupled to the input interface 912 and the output interface 916 for example via buses or other connections such that received data may be processed by the data processor and subsequently processed data (e.g. including the adherence levels) may be output via the output interface. The input and output interfaces are capable of receiving for example electrical or optical signals representative of or comprising the necessary and described data and the data processor may be capable of processing such electrical signals and/or optical signals to perform the steps described herein for manipulating the received data to provide the result data.

The data processor 914 is configured to generate the adherence data from input data by performing the methods or steps as described herein for example with reference to flowcharts as described herein. In embodiments the processor is configured determine motion vectors (202) corresponding to the lead (104) from the data corresponding to the blood vessel (102) and to input the motion vectors (202) into a machine learning algorithm (302). The data processor configured to use the machine learning algorithm trained to learn the correlation between the motion vectors (202) and whether a section of a lead (104) has adhered to a blood vessel (102), is then able to output an adherence level (310) for one or more segments (304, 306, 308) of the lead (104). The adherence levels are then provided or sent to the output interface 916. The data processor may be further configured to have the machine learning algorithm (302) output a position of adhesion based on the segments (304, 306, 308) and their respective adherence level (310). The data processor may be configured to have the machine learning algorithm (302) output a suggested viewing angle, wherein an image of the blood vessel (102) taken at the suggested viewing angle is likely to provide accurate motion vectors (202).

In some embodiments the data processor is configured to perform a machine learning algorithm (302) based on one or more of: a convolutional neural network; a multi-layer perceptron; a support vector machine; a decision tree model; multivariate regression model.

In some embodiments the system or data processor may be configured to generated and output to the user interface the adherence level (310) with the corresponding motion vectors (202) overlaid on or together with images of the blood vessel (102).

In some embodiments the motion vectors (202) are 2D motion vectors.

In some embodiments the input is for receiving the data corresponding to the blood vessel (102) such that it comprises or consists of two or more images and preferably two or more 2D images obtained via, for example, 2D fluoroscopy and/or 2D venography.

In some embodiments also the data corresponding to the blood vessel (102) are input to the machine learning algorithm.

The output interface 916 is configured to provide data or a signal representative of such data where the data comprise the adherence level 310 and where the signal or the data are suitable to be received by a user interface such as for example one comprising a display for displaying the adherence data. The output of data 310 and possible other data is indicated with a first hashed arrow 920.

A frame 924 indicates the option to provide the input interface 912, the data processor 914 and the output interface 16 in an integrated manner, for example in a common housing of e.g a workstation or imaging system. In an option, the input interface 912, the data processor 914 and the output interface 16 are provided as separate arrangements as described herein before.

The data received or obtained by the system preferably relates to a supply of image data. The input interface 912 then provides an interface for inputting image data. The input interface 912 can also be referred to as image data source, image data supply, data input, data supply unit, data input unit or data supply module. The input interface 912 can be connected to a data storage or to an imaging source like in stand-alone fashion or connected to an X-ray imaging arrangement.

The input interface 912 provides the data to the data processor 914. The term "receive" relates to a (for example, passive) provision of data to the system such that feeds the data to the data processor 14. Alternatively, also the term "obtain" could be used that relates to a (for example, active) behavior of the system where e.g. the processor makes or causes the input interface 912 to receive the data from a source such as a storage or imaging system or imaging device.

In some embodiments the "processor" or "data processor" relates to image data processing. The data processor 914 can also be referred to as processor, processing unit or computing module.

The "output interface" relates to the provision of the result of the data processing. The output interface 916 can also be referred to as output unit or output module.

As an effect, the provision of the adherence data provides an improved knowledge about the lead adherence situation in a subject either before or during a lead removal procedure. It is thus also possible to conduct intervention assessments, e.g. by comparing the situation before and after a treatment and/or monitoring a situation during a treatment.

The processor 914 may include a central processing unit (CPU), a digital signal processor (DSP), an ASIC, a controller, an FPGA, another hardware device, a firmware device, or any combination thereof configured to perform the operations described herein. The processor 914 may also be implemented as a combination of computing devices, e.g., a combination of a DSP and a microprocessor, a plurality of microprocessors, one or more microprocessors in conjunction with a DSP core, or any other such configuration.

The memory 926 may include a cache memory (e.g., a cache memory of the processor 914), random access memory (RAM), magnetoresistive RAM (MRAM), read-only memory (ROM), programmable read-only memory (PROM), erasable programmable read only memory (EPROM), electrically erasable programmable read only memory (EEPROM), flash memory, solid state memory device, hard disk drives, other forms of volatile and nonvolatile memory, or a combination of different types of memory. In an embodiment, the memory 926 includes a non-transitory computer-readable medium. The memory and/or non-transitory computer-readable medium may store instructions. For example, the memory 926, or non-transitory computer-readable medium may have program code recorded thereon, the program code including instructions for causing the system to perform its functions or for causing the processor to perform the described operations for executing the methods in a computer implemented fashion. For example, the processor circuit 914 can execute one or more operations of the methods described herein. Instructions may also be referred to as code or program code. The terms "instructions" and "code" should be interpreted broadly to include any type of computer-readable statement(s). For example, the terms "instructions" and "code" may refer to one or more programs, routines, sub-routines, functions, procedures, etc. "Instructions" and "code" may include a single computer-readable statement or many computer-readable statements. The memory 964, with the code recorded thereon, may be referred to as a computer program product.

The processing system or processor may be a single processor or a distributed processor comprising multiple separate processors configured to communicate with each other via busses and or network connections such as LAN or WLAN. The processing system may take the form of a single device or apparatuses that is connectable to an imaging apparatus. For example, such single device may comprise or be a workstation. As mentioned in some embodiments, the processor circuit and/or the imaging system has a remotely distributed form with the distributed elements interconnected or interconnectable via a data transfer network such as LAN or WLAN.

A computer program may be stored/distributed on a suitable medium, such as an optical storage medium or a solid-state medium supplied together with or as part of other hardware, but may also be distributed in other forms, such as via the Internet or other wired or wireless telecommunication systems.

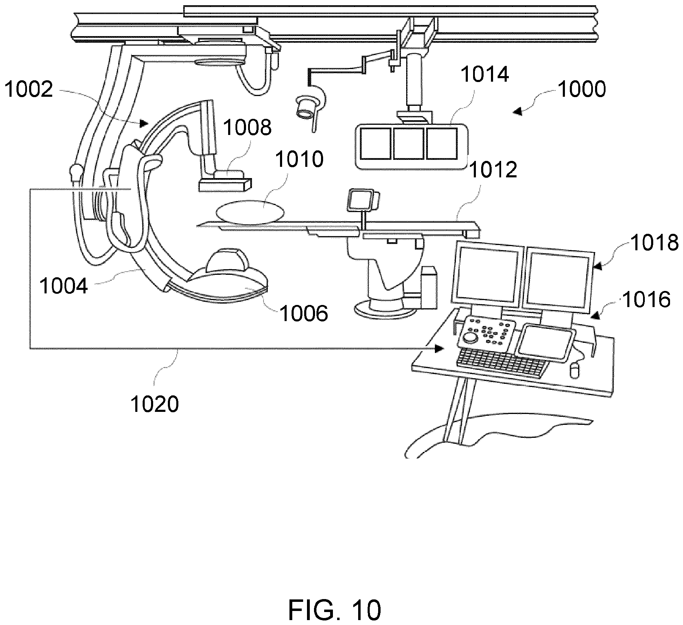

Figure 10 shows an example of an imaging system 1000 comprising the system of Figure 9. The system 1000 comprises an X-ray imaging device 1002. As an example, the X-ray imaging device 1002 is shown as a ceiling mounted movable C-arm structure 1004 with an X-ray source 1006 and an X-ray detector 1008 attached to ends of the C-arm. For illustration purposes, also an object 1010, i.e. a subject, is shown supported by a patient support 1012 for examination, intervention, other treatment or imaging purposes. The system 1000 further comprises a display device 1014, e.g. in form of a monitor arrangement shown movably suspended from a ceiling. The system 1000 also comprises a system 1016 for for determining whether a section of a lead has adhered to a blood vessel according to one of the preceding and following examples. Further, again for illustration purposes, a control console with a monitor 1018 and other user interface equipment like a mouse, a keyboard, a control panel and a touchpad is indicated on the right side. To this end the system 900 can include appropriate input and communication devices as well as the corresponding software to provide such functionality. The device 1016 may be integrated in the control console. A line 1020 indicates the data connection (wire or wireless) of the X-ray imaging device 1002 and the device 1016. This connection would for example enable communication of the data from the imaging system or its storage device to the system of Figure 9. The X-ray imaging device 1002 is configured to provide X-ray images of a region of interest of the subject's body such as that where the leads are located. The display device 1014 is configured to display the macrovascular-related time density curve and the microvascular-related time density curve. The imaging system is known in the art and out on the market for sale and will not be described in further detail.

The mere fact that certain measures are recited in mutually different dependent claims does not indicate that a combination of these measures cannot be used to advantage.

If the term "adapted to" is used in the claims or description, it is noted the term "adapted to" is intended to be equivalent to the term "configured to".

Any reference signs in the claims should not be construed as limiting the scope.

Variations to the disclosed embodiments can be understood and effected by those skilled in the art in practicing the present disclosure, from a study of the drawings, the disclosure and the appended claims. In the claims, the word "comprising" does not exclude other elements or steps, and the indefinite article "a" or "an" does not exclude a plurality.

## Claims

1. A method for determining whether a section of a lead (104) has adhered to a blood vessel (102), the method comprising:
receiving or obtaining data corresponding to the blood vessel (102), wherein the blood vessel (102) has a lead (104) inside;
determining motion vectors (202) corresponding to the lead (104) from the data corresponding to the blood vessel (102);
inputting the motion vectors (202) into a machine learning algorithm (302), wherein:
the machine learning algorithm (302) is trained to learn the correlation between the motion vectors (202) and whether a section of a lead (104) has adhered to a blood vessel (102), and
the machine learning algorithm (302) is configured to output an adherence level (310) for segments (304, 306, 308) of the lead (104) based on the input motion vectors, and
optionally, providing the adherence level to a user.

2. The method of claim 1, wherein the motion vectors (202) are 2D motion vectors.

3. The method of claim any one of claims 1 or 2, wherein the data corresponding to the blood vessel (102) comprises or consists of two or more images and preferably two or more 2D images obtained via, for example, 2D fluoroscopy and/or 2D venography.

4. The method of any one of claims 1 to 3, wherein the machine learning algorithm (302) is further configured to output a position of adhesion based on the segments (304, 306, 308) and their respective adherence level (310).

5. The method of any one of claims 1 to 4, wherein further inputs to the machine learning algorithm (302) are the data corresponding to the blood vessel (102).

6. The method of any one of claims 1 to 5, wherein the machine learning algorithm (302) is further configured to output a suggested viewing angle, wherein an image of the blood vessel (102) taken at the suggested viewing angle is likely to provide accurate motion vectors (202).

7. The method of any one of claims 1 to 6, wherein the machine learning algorithm (302) is based on:
a convolutional neural network;
a multi-layer perceptron;
a support vector machine;
a decision tree model;
multivariate regression model; or
a combination thereof.

8. The method of any one of claims 1 to 7, further comprising displaying the adherence level (310) with the corresponding motion vectors (202) on images of the blood vessel (102).

9. The method of any one of claims 1 to 7, wherein the adherence level (310) is based on a binary classification or an adhesion range.

10. A computer program product comprising computer program code means which, when executed on a computing device having a processing system, cause the processing system to perform all of the steps of the method according to any of claims 1 to 9.

11. A system (900) for determining whether a section of a lead (104) has adhered to a blood vessel (102), the system comprising:
an input interface (912) configured to receive or obtain data (922) corresponding to the blood vessel (102), wherein the blood vessel (102) has a lead (104) inside; and
a processor (914) configured to:
determine motion vectors (202) corresponding to the lead (104) from the data corresponding to the blood vessel (102);
input the motion vectors (202) into a machine learning algorithm (302), wherein:
the machine learning algorithm (302) is trained to learn the correlation between the motion vectors (302) and whether a section of a lead (104) has adhered to a blood vessel (102), and
the machine learning algorithm (302) is configured to output an adherence level (310) for segments (304, 306, 308) of the lead (104), and
an output interface (916) configured to provide the adherence level (920) to a user interface (918), and
optionally, the output interface (918) for providing the adherence level to a user.

12. The system of claim 11, further comprising an imaging device configured to obtain images of the blood vessel (102) and send data (922) representative of the images to the input interface.

13. The system of claim 12, wherein the imaging device is a 2D imaging device configured to obtain 2D images, for example for obtaining 2D fluoroscopic images and/or 2D venography images.

14. The system of any one of claims 12 to 13, wherein the user interface further comprises a display configured to display the images of the blood vessel (102), the motion vectors (202) and the corresponding adherence level (310).

15. A method for training a machine learning algorithm to learn the correlation between motion vectors corresponding to a lead inside a blood vessel and whether a section of the lead has adhered to the blood vessel, the method comprising:
obtaining a first set of data corresponding to a blood vessel with a lead inside;
determining a ground truth for the first set of data corresponding to the blood vessel, wherein the ground truth defines whether the lead has adhered to the blood vessel and the location of the adhesion;
obtaining a second set of data corresponding to the blood vessel with a lead inside, wherein the second set of data is obtained at the same anatomical locations as the first set of data within the blood vessel;
determining motion vectors corresponding to the lead from the second set of data corresponding to the blood vessel;
inputting the motion vectors into the machine learning algorithm, wherein the machine learning algorithm is configured to output an adherence level for segments of the lead;
comparing the adherence levels output by the machine learning algorithm to the corresponding ground truth and determining an error based on the comparison between the adherence levels and the ground truth; and
adapting parameters of the machine learning algorithm based on the error.
